# EUROPEAN PATENT SPECIFICATION

(11) **EP 4 580 606 B1**
(45) Date of publication and mention of the grant of the patent: **27.05.2026**
(21) Application number: 23764853.0
(22) Date of filing: 30.08.2023
(51) Int. Cl.: A61K 9/20, A61K 9/28, A61K 31/519, A61P 9/00

(54) **PHARMACEUTICAL FORMULATION**
PHARMAZEUTISCHE FORMULIERUNG
FORMULATION PHARMACEUTIQUE

(30) Priority: 31.08.2022 WO PCT/CN2022/116379
(43) Date of publication of application: 09.07.2025
(73) Proprietor: Astrazeneca AB, 151 85 Södertälje (SE)
(72) Inventor: DENG, Wanding, Shanghai 201203 (CN); FAN, Ying, Shanghai 201203 (CN)
(74) Representative: J A Kemp LLP
(86) International application number: PCT/EP2023/073764
(87) International publication number: WO 2024/047094

(56) References cited:
- WO-A1-2016/087338
- INGHARDT TORD ET AL: "Discovery of AZD4831, a Mechanism-Based Irreversible Inhibitor of Myeloperoxidase, As a Potential Treatment for Heart Failure with Preserved Ejection Fraction", JOURNAL OF MEDICINAL CHEMISTRY, vol. 65, no. 17, 25 August 2022 (2022-08-25), US, pages 11485 - 11496, XP093108430, ISSN: 0022-2623, Retrieved from the Internet <URL:https://pubs.acs.org/doi/pdf/10.1021/acs.jmedchem.1c02141> [retrieved on 20231204], DOI: 10.1021/acs.jmedchem.1c02141

## Description

### FIELD

The present specification relates to a pharmaceutical formulation comprising 1-{2-[(1R)-1-aminoethyl]-4-chlorobenzyl }-2-thioxo-1,2,3,5-tetrahydro-4H-pyrrolo[3,2-d]pyrimidin-4-one (Compound 1). The specification also relates to the use of the formulation in therapy, particularly the treatment of heart failure with preserved ejection fraction (HFpEF) and chronic obstructive pulmonary disease (COPD), as well as methods of treatment involving the administration of the formulation.

### BACKGROUND

1-{2-[(1R)-1-aminoethyl]-4-chlorobenzyl}-2-thioxo-1,2,3,5-tetrahydro-4H-pyrrolo[3,2-d]pyrimidin-4-one (Compound 1 (below)) is disclosed in international patent application WO2016/087338.

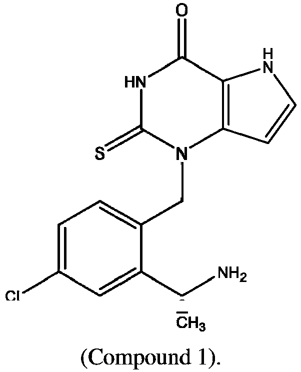

Compound 1 is very adhesive and easily agglomerates into large (0.5 - 1mm) particles. A suitable formulation must have good manufacturability (the extent to which a product can be manufactured with relative ease at minimum cost and maximum reliability) because a robust process is needed to deliver product for late-stage clinical trials and commercial purposes. Where the formulation is a tablet, the tablets must achieve a suitable tensile strength, without the need for the application of a high compression force, whilst maintaining an acceptable tablet porosity. These factors minimise the risk of over-compression and variable dissolution rates - a suitable formulation must release its active ingredient in a uniform and acceptable manner. Finally, a formulation for human use must employ pharmaceutically acceptable excipients that meet regulatory requirements for chronic dosing, and must be stable for a meaningful period of time, typically > 1 year and ideally up to 3 years.

The present specification describes a pharmaceutical formulation of Compound 1 that satisfies these criteria and is suitable for manufacture on a commercial scale.

### DETAILED DESCRIPTION

The scope of the invention is defined by the appended claims. Any reference herein to methods of treatment is to be interpreted as a reference to the compositions for use in a method of treatment according to the present invention. Methods of medical treatment do not form part of the invention.

"A" means "at least one". In any embodiment where "a" is used to denote a given material or element, "a" may mean one.

"Comprising" means that a given material or element may contain other materials or elements. In any embodiment where "comprising" is mentioned the given material or element may be formed of at least 10% w/w, at least 20% w/w, at least 30% w/w, or at least 40% w/w of the material or element. In any embodiment where "comprising" is mentioned, "comprising" may also mean "consisting of" (or "consists of") or "consisting essentially of" (or "consists essentially of') a given material or element.

"Consisting of" or "consists of" means that a given material or element is formed entirely of the material or element. In any embodiment where "consisting of" or "consists of" is mentioned the given material or element may be formed of 100% w/w of the material or element.

"Consisting essentially of" or "consists essentially of" means that a given material or element consists almost entirely of that material or element. In any embodiment where "consisting essentially of" or "consists essentially of" is mentioned the given material or element may be formed of at least 50% w/w, at least 60% w/w, at least 70% w/w, at least 80% w/w, at least 90% w/w, at least 95% w/w or at least 99% w/w of the material or element.

"w/w" means weight by weight.

In any embodiment where "is" or "may be" is used to define a material or element, "is" or "may be" may mean the material or element "consists of" or "consists essentially of" the material or element.

### COMPOUND 1

Compound 1, is 1-{2-[(1R)-1-aminoethyl]-4-chlorobenzyl}-2-thioxo-1,2,3,5-tetrahydro-4H-pyrrolo[3,2-d]pyrimidin-4-one (disclosed in international patent application WO2016/087338).

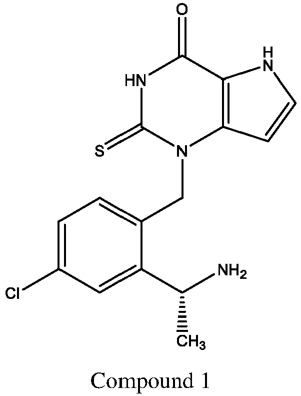

In some embodiments, Compound 1 refers to free base Compound 1.

In some embodiments, Compound 1 refers to a pharmaceutically-acceptable salt of Compound 1.

In some embodiments, the pharmaceutical formulation described herein comprises 1-10% w/w Compound 1.

In some embodiments, the pharmaceutical formulation described herein comprises 10% w/w Compound 1.

In some embodiments, the pharmaceutical formulation described herein comprises 5% w/w Compound 1.

In some embodiments, the pharmaceutical formulation described herein comprises 2.5 %w/w Compound 1.

### FILLERS

In some embodiments, the pharmaceutical formulation described herein comprises one or more pharmaceutically acceptable fillers. In further embodiments, the one or more pharmaceutically acceptable fillers are selected from mannitol, microcrystalline cellulose, and dibasic calcium phosphate anhydrous.

In certain embodiments, the pharmaceutical formulation described herein comprises about 80-95% w/w of one or more pharmaceutically acceptable fillers, including all values and subranges there between, such as, about 80-85% w/w; about 80-90% w/w; about 80-92%; about 82-87% w/w; about 82-90% w/w; about 82-92% w/w; about 82-95% w/w; about 85-87% w/w; about 85-90% w/w; about 85-92% w/w; about 85-95% w/w; about 87-90% w/w; about 87-92% w/w; about 87-95% w/w; about 89-92% w/w; about 89-95% w/w; about 91-93% w/w; about 91-95%, about 83%, about 84%, about 85% w/w, about 86% w/w, about 87% w/w, about 88% w/w, about 89% w/w, about 90% w/w, about 91% w/w, about 92% w/w, about 93% w/w, about 94% w/w, and about 95% w/w.

### Mannitol

Mannitol is a polyol (sugar alcohol) with the chemical formula: C₆H₁₄O₆. Mannitol can be used as a filler.

In some embodiments, the pharmaceutical formulation described herein comprises mannitol with an average mean particle diameter between about 50 and 300 µm.

In some embodiments, the pharmaceutical formulation described herein comprises mannitol with an average mean particle diameter between about 100 and 200 µm.

In some embodiments, the pharmaceutical formulation described herein comprises mannitol with an average mean particle diameter of about 100 µm.

In some embodiments, the pharmaceutical formulation described herein comprises mannitol with an average mean particle diameter of about 200 µm.

In certain embodiments, the pharmaceutical formulation described herein comprises about 20-75% w/w of mannitol, including all values and subranges there between, such as, 20-30% w/w; 20-40% w/w; 20-50% w/w; 20-60% w/w; 20-70% w/w; 30-40% w/w; 30-50% w/w; 30-60% w/w; 30-75% w/w; 40-50% w/w; 40-60% w/w; 40-75% w/w; 50-60% w/w; 50-75% w/w; 60-75% w/w; and 65-75% w/w.

In certain embodiments, the pharmaceutical formulation described herein comprises about 55% w/w of mannitol. In some embodiments, the pharmaceutical formulation described herein comprises about 55% w/w of mannitol with an average mean particle diameter between about 50 and 300 µm. In some embodiments, the pharmaceutical formulation described herein comprises about 55% w/w of mannitol with an average mean particle diameter between about 100 and 200 µm. In some embodiments, the pharmaceutical formulation described herein comprises about 55% w/w of mannitol with an average mean particle diameter of about 200 µm. In some embodiments, the pharmaceutical formulation described herein comprises about 55% w/w of mannitol with an average mean particle diameter of about 100 µm.

In certain embodiments, the pharmaceutical formulation described herein comprises 55% w/w of mannitol. In some embodiments, the pharmaceutical formulation described herein comprises 55% w/w of mannitol with an average mean particle diameter between about 50 and 300 µm. In some embodiments, the pharmaceutical formulation described herein comprises 55% w/w of mannitol with an average mean particle diameter between about 100 and 200 µm. In some embodiments, the pharmaceutical formulation described herein comprises 55% w/w of mannitol with an average mean particle diameter of about 200 µm. In some embodiments, the pharmaceutical formulation described herein comprises 55% w/w of mannitol with an average mean particle diameter of about 100 µm.

### Microcrystalline cellulose

Microcrystalline cellulose (MCC) can also be used as filler. Microcrystalline cellulose is available in different grades and particle sizes. It may also be referred to as powdered cellulose, cellulose gum or carboxymethylcellulose.

In some embodiments microcrystalline cellulose refers to microcrystalline cellulose with a median particle size of about 40-200µM.

In some embodiments microcrystalline cellulose refers to microcrystalline cellulose with a median particle size of about 80-120µM.

In some embodiments microcrystalline cellulose refers to microcrystalline cellulose with a median particle size of about 100µM.

In some embodiments microcrystalline cellulose refers to Avicel^{®} PH-102 microcrystalline cellulose.

In some embodiments, the pharmaceutical formulation described herein comprises 20-50% w/w microcrystalline cellulose.

In some embodiments, the pharmaceutical formulation described herein comprises 30-40% w/w microcrystalline cellulose.

In some embodiments, the pharmaceutical formulation described herein comprises 30-36% w/w microcrystalline cellulose.

In some embodiments, the pharmaceutical formulation described herein comprises 33-34% w/w microcrystalline cellulose.

In some embodiments, the pharmaceutical formulation described herein comprises about 33.5% w/w microcrystalline cellulose.

In some embodiments, the pharmaceutical formulation described herein comprises 33.5% w/w microcrystalline cellulose.

In some embodiments microcrystalline cellulose refers to cellulose, microcrystalline of European Pharmacopoeial standard.

In some embodiments microcrystalline cellulose refers to microcrystalline cellulose of Japanese Pharmacopoeial standard.

In some embodiments microcrystalline cellulose refers to microcrystalline cellulose of United States Pharmacopeial standard.

### Dibasic Calcium Phosphate Anhydrous (DCPA)

Dibasic calcium phosphate anhydrous (DCPA) has the chemical formula: CaHPO₄. DCPA can be used as a water insoluble filler. DCPA is also known as calcium hydrogen phosphate and phosphoric acid calcium salt (1:1).

In some embodiments, the pharmaceutical formulation described herein comprises about 5-40% w/w anhydrous dibasic calcium phosphate.

In some embodiments, the pharmaceutical formulation described herein comprises about 5-15% w/w anhydrous dibasic calcium phosphate.

In some embodiments, the pharmaceutical formulation described herein comprises about 10% w/w anhydrous dibasic calcium phosphate.

In some embodiments, the pharmaceutical formulation described herein comprises about 20-30% w/w anhydrous dibasic calcium phosphate.

In some embodiments, the pharmaceutical formulation described herein comprises about 25% w/w anhydrous dibasic calcium phosphate.

In some embodiments, the pharmaceutical formulation described herein comprises about 30-40% w/w anhydrous dibasic calcium phosphate.

In some embodiments, the pharmaceutical formulation described herein comprises about 36.5% w/w anhydrous dibasic calcium phosphate.

In some embodiments, the pharmaceutical formulation described herein comprises 36.5% w/w anhydrous dibasic calcium phosphate.

In some embodiments dibasic calcium phosphate anhydrous refers to dibasic calcium phosphate anhydrous of European Pharmacopoeial standard.

In some embodiments dibasic calcium phosphate refers anhydrous to dibasic calcium phosphate anhydrous of Japanese Pharmacopoeial standard.

In some embodiments dibasic calcium phosphate anhydrous refers to dibasic calcium phosphate anhydrous of United States Pharmacopeial standard.

### DISINTEGRANTS

In some embodiments, the pharmaceutical formulation described herein comprises one or more pharmaceutically acceptable disintegrants.

In some embodiments, the pharmaceutical formulation described herein comprises one or more pharmaceutically acceptable disintegrants selected from croscarmellose sodium and low substituted hydroxypropyl cellulose.

In some embodiments, the pharmaceutical formulation described herein comprises croscarmellose sodium.

In some embodiments, the pharmaceutical formulation described herein comprises low substituted hydroxypropyl cellulose.

### Croscarmellose Sodium

Croscarmellose sodium, or sodium CMC, is a cross-linked polymer of carboxymethylcellulose sodium.

In some embodiments, the pharmaceutical formulation described herein comprises 1-10% w/w croscarmellose sodium.

In some embodiments, the pharmaceutical formulation described herein comprises 3-5% w/w croscarmellose sodium.

In some embodiments, the pharmaceutical formulation described herein comprises about 4% w/w croscarmellose sodium.

In some embodiments, the pharmaceutical formulation described herein comprises 4% w/w croscarmellose sodium.

### Low-Substituted Hydroxypropyl Cellulose

Hydroxypropyl cellulose (HPC) is an ether of cellulose in which some of the hydroxyl groups in the repeating glucose units have been hydroxypropylated forming -OCH₂CH(OH)CH₃ groups. L-HPC is a low-substituted hydroxypropyl ether of cellulose within a small portion of the hydroxypropyl groups in the glucose unit. Typically, L-HPC has a molar substitution of 0.2-0.4. L-HPC is available in various grades, including LH-11, LH-21, LH-22, LH-31, and LH-B1.

In some embodiments, the pharmaceutical formulation described herein comprises 5-20% w/w low substituted hydroxypropyl cellulose.

In some embodiments, the pharmaceutical formulation described herein comprises 7.5-12.5% w/w low substituted hydroxypropyl cellulose.

In some embodiments, the pharmaceutical formulation described herein comprises about 10% w/w low substituted hydroxypropyl cellulose.

In some embodiments, the pharmaceutical formulation described herein comprises 10% w/w low substituted hydroxypropyl cellulose.

In some embodiments, the low substituted hydroxypropyl cellulose is LH-21 grade low substituted hydroxypropyl cellulose.

In some embodiments, the low substituted hydroxypropyl cellulose is LH-31 grade low substituted hydroxypropyl cellulose.

In some embodiments, the low substituted hydroxypropyl cellulose is LH-B1 grade low substituted hydroxypropyl cellulose.

In some embodiments low substituted hydroxypropyl cellulose refers to hydroxypropyl cellulose, low-substituted of European Pharmacopoeial standard.

In some embodiments low substituted hydroxypropyl cellulose refers to low-substituted hydroxypropyl cellulose of National Formulary standard.

In some embodiments low substituted hydroxypropyl cellulose refers to low substituted hydroxypropyl cellulose of Japanese Pharmacopoeial standard.

### LUBRICANTS

### Magnesium stearate

Magnesium stearate has the formula Mg(C₁₈H₃₅O₂)₂. It is used as a lubricant to reduce friction and is typically a white, water-insoluble powder.

In some embodiments, the pharmaceutical formulation described herein comprises 1-3% w/w magnesium stearate.

In some embodiments, the pharmaceutical formulation described herein comprises about 2% w/w magnesium stearate.

In some embodiments, the pharmaceutical formulation described herein comprises 2% w/w magnesium stearate.

In some embodiments magnesium stearate refers to magnesium stearate of United States Pharmacopoeial standard.

In some embodiments magnesium stearate refers to magnesium stearate of British Pharmacopoeial standard.

In some embodiments magnesium stearate refers to magnesium stearate of European Pharmacopoeial standard.

In some embodiments magnesium stearate refers to magnesium stearate of Japanese Pharmacopoeial standard.

In some embodiments magnesium stearate refers to magnesium stearate of Chinese Pharmacopoeial standard.

### Pharmacopoeia

Herein where pharmacopeia standards are mentioned, it is to be understood that the standards are as published in the following editions of the pharmacopoeias:

**Table 1: Pharmacopeia Editions**

| **Pharmacopoeia** | **Edition** |
|---|---|
| United States Pharmacopoeia | 43 |
| British Pharmacopoeia | 2020 Edition |
| European Pharmacopoeia | 10^{th} Edition |
| Japanese Pharmacopoeia | 17^{th} Edition |
| Chinese Pharmacopoeia | 2020 Edition |
| National Formulary | 38 |

### TABLET

In some embodiments, the pharmaceutical formulation described herein may be a tablet.

In some embodiments, the pharmaceutical formulation described herein may be a tablet for oral administration.

In some embodiments, the pharmaceutical formulation described herein may be an immediate release tablet.

In some embodiments, the pharmaceutical formulation described herein may be administered daily.

In some embodiments, the pharmaceutical formulation described herein may be a tablet administered daily.

In some embodiments, the pharmaceutical formulation described herein may be administered orally.

In some embodiments, the pharmaceutical formulation described herein may be a tablet administered orally.

In some embodiments, the pharmaceutical formulation described herein may comprise 1 to 10 mg of Compound 1.

In some embodiments, the pharmaceutical formulation described herein may comprise 10 mg of Compound 1.

In some embodiments, the pharmaceutical formulation described herein may comprise 5 mg of Compound 1.

In some embodiments, the pharmaceutical formulation described herein may comprise 2.5 mg of Compound 1.

In some embodiments, the pharmaceutical formulation described herein may be a tablet comprising 2.5mg of Compound 1.

In some embodiments, the pharmaceutical formulation described herein may be a tablet comprising 5mg of Compound 1.

In some embodiments, the pharmaceutical formulation described herein may be a tablet comprising 10mg of Compound 1.

In some embodiments, the pharmaceutical formulation described herein may comprise:
About 2.5% w/w Compound 1
About 55% w/w mannitol
About 36.5% w/w microcrystalline cellulose
About 4% w/w croscarmellose sodium
About 2% w/w magnesium stearate.

In some embodiments, the pharmaceutical formulation described herein may comprise:
About 2.5% w/w Compound 1
About 55% w/w mannitol
About 36.5% microcrystalline cellulose
About 4% w/w croscarmellose sodium
About 2% magnesium stearate.

In some embodiments, the pharmaceutical formulation described herein may comprise:
1-10% w/w Compound 1;
45-60% w/w mannitol;
30-40% w/w microcrystalline cellulose;
3-5% w/w croscarmellose sodium; and
1-3 % w/w magnesium stearate.

In some embodiments, the pharmaceutical formulation described herein may comprise:
2.5% w/w Compound 1;
55% w/w mannitol;
36.5% w/w microcrystalline cellulose;
4% w/w croscarmellose sodium; and
2% w/w magnesium stearate.

In some embodiments, the pharmaceutical formulation described herein may comprise:
2.5% w/w Compound 1;
55% w/w mannitol having an average mean particle diameter of about 100 µm. ;
36.5% w/w microcrystalline cellulose having a median particle size of about 100µM.;
4% w/w croscarmellose sodium; and
2% w/w magnesium stearate.

In some embodiments, the pharmaceutical formulation described herein may comprise:
2.5 mg Compound 1;
55 mg mannitol;
36.5 mg microcrystalline cellulose;
4.0 mg croscarmellose sodium; and
2.0 mg magnesium stearate.

In some embodiments, the pharmaceutical formulation described herein may comprise:
5.0 mg Compound 1;
110.0 mg mannitol;
73.0 mg microcrystalline cellulose;
8.0 mg croscarmellose sodium; and
4.0 mg magnesium stearate.

### COATINGS

In some embodiments, where the pharmaceutical formulation is in the form of a tablet, it may be advantageous to coat the tablet with a coating. The resultant coated tablet would then comprise a tablet core and a coating. A coated tablet may assist a patient in swallowing a tablet. A coloured coating may assist with patient compliance and/or differentiation from other medicines, particularly those administered as part of a combination treatment.

In some embodiments, the pharmaceutical formulation described herein may be a coated tablet. If the tablet is a coated tablet it is to be understood that any % w/w value quoted herein refers to the w/w composition of the tablet core.

In some embodiments, the pharmaceutical formulation described herein may be a colour coated tablet.

In some embodiments, the pharmaceutical formulation described herein may be a film-coated tablet.

In some embodiments, the pharmaceutical formulation described herein may be a tablet coated with a film-coating comprising a film forming polymer. In further embodiments, the film forming polymer is selected from hydroxypropyl methylcellulose (hypromellose, HPMC), hydroxypropyl cellulose (HPC), polyvinylpyrrolidone (PVP), polyvinylpolypyrrolidone (PVPP), and mixtures thereof. In further embodiments, the film forming polymer is selected from HPMC, HPC, and mixtures thereof. In still further embodiments, the film forming polymer is HPMC.

In still further embodiments, the film-coating further comprises a pigment, such as titanium dioxide, iron oxide yellow, iron oxide red, ferrosoferric oxide, and mixtures thereof.

In some embodiments, the pharmaceutical formulation described herein may be a coloured film-coated tablet.

In some embodiments, the pharmaceutical formulation described herein may be a white film-coated tablet.

In some embodiments, the pharmaceutical formulation described herein may be an orange film-coated tablet.

In some embodiments, the coating is present at about 1.5 - 4.5% w/w, including all values and subranges there between, including about 1.5-3.5% w/w, 1.5-2.5% w/w, 2-4% w/w, 2-3% w/w, 2.5 - 4.5% w/w, 2.5-3.5% w/w, 3-4% w/w, 2.4% w/w, 2.5% w/w, 2.6% w/w, 2.7%, 2.8% w/w, 2.9% w/w, 3% w/w, 3.1% w/w, 3.2% w/w, 3.3% w/w, 3.4% w/w, 3.5% w/w, 3.6% w/w, 3.7% w/w, 3.9% w/w, 3.9% w/w, and 4% w/w.

### METHOD OF TREATMENT

Prophylaxis of diseases or conditions in which modulation of the activity of the enzyme myeloperoxidase (MPO) is desirable. In particular, linkage of MPO activity to disease has been implicated in numerous diseases, including diseases with inflammatory, cardiovascular, and/or neurodegenerative components, as well as neutrophilic driven diseases.

In some embodiments there is provided a pharmaceutical formulation as disclosed herein, for use in a method of treatment of the human or animal body by therapy.

In some embodiments there is provided a method of treating diseases or conditions in which modulation of the activity of the enzyme myeloperoxidase (MPO) is desirable in a warm-blooded animal, such as man, which comprises administering to said animal an effective amount of a pharmaceutical formulation described herein.

In some embodiments there is provided a method of treating diseases or conditions in which inhibition of the activity of the enzyme myeloperoxidase (MPO) is desirable in a warm-blooded animal, such as man, which comprises administering to said animal an effective amount of a pharmaceutical formulation described herein.

In some embodiments there is provided a method of treating diseases or conditions with inflammatory, cardiovascular, and/or neurological components, and/or neutrophilic driven diseases in a warm-blooded animal, such as man, which comprises administering to said animal an effective amount of a pharmaceutical formulation described herein.

In some embodiments there is provided a method of treating autoimmune diseases, chronic kidney disease (CKD), acute kidney injury (AKI), renal glomerular damage, nephritis, glomerulonephritis, interstitial nephritis, tubulointerstitial nephritis, diabetic nephropathy, cardiorenal syndrome (CRS), non-alcoholic steatohepatitis (NASH), non-alcoholic fatty liver disease (NAFLD), inflammatory bowel disease (IBD), Crohn's disease, colitis, ulcerative colitis, irritable bowel syndrome (IBS), rheumatoid arthritis, systemic lupus erythematosus, liver steatosis, liver fibrosis, gout, sickle cell disease, cystic fibrosis, vasculitis, anti-neutrophilic cytoplasmic autoantibody (ANCA)-associated vasculitis, asthma, chronic obstructive pulmonary disease (COPD), non-cystic fibrosis bronchiectasis (NCFB), vascular dysfunction, lipoprotein modification, and/or type 2 diabetes in a warm-blooded animal, such as man, which comprises administering to said animal an effective amount of a pharmaceutical formulation described herein.

In some embodiments there is provided a method of treating coronary artery disease, acute coronary syndrome, heart failure, heart failure with reduced ejection fraction (HFrEF), heart failure with preserved ejection fraction (HFpEF), arrhythmia, cardiomyopathy, dilated cardiomyopathy, hypertrophic cardiomyopathy myocardial infarction, hypertension, pulmonary arterial hypertension (PAH), pulmonary hypertension, vascular dysfunction, atherosclerosis, ischemic heart disease, atrial fibrillation, pericarditis, diastolic dysfunction, atherosclerotic plaque rupture, abdominal aortic aneurysm, and/or chemotherapy-induced cardiotoxicity in a warm-blooded animal, such as man, which comprises administering to said animal an effective amount of a pharmaceutical formulation described herein.

In some embodiments there is provided a method of treating Alzheimer's disease, Parkinson's disease, stroke, multiple sclerosis (MS), multiple system atrophy (MSA), amyotrophic lateral sclerosis (ALS), and /or epilepsy in a warm-blooded animal, such as man, which comprises administering to said animal an effective amount of a pharmaceutical formulation described herein.

In some embodiments there is provided a method of treating heart failure, heart failure with reduced ejection fraction, heart failure with preserved ejection fraction, non-alcoholic steatohepatitis (NASH), or chronic obstructive pulmonary disease (COPD) in a warm-blooded animal, such as man, which comprises administering to said animal an effective amount of a pharmaceutical formulation described herein.

In some embodiments, there is provided a pharmaceutical formulation as described herein comprising Compound 1 for use as a medicament.

In some embodiments there is provided a pharmaceutical formulation as described herein, for use in therapy.

In some embodiments, there is provided the use of a pharmaceutical formulation as described herein, in the manufacture of a medicament for treating diseases or conditions in which modulation of the activity of the enzyme myeloperoxidase (MPO) is desirable.

In some embodiments, there is provided the use of a pharmaceutical formulation as described herein, in the manufacture of a medicament for treating diseases or conditions in which inhibition of the activity of the enzyme myeloperoxidase (MPO) is desirable.

In some embodiments there is provided the use of a pharmaceutical formulation as described herein, in the manufacture of a medicament for use in treating diseases or conditions with inflammatory, cardiovascular, and/or neurological components, and/or neutrophilic driven diseases.

In some embodiments there is provided the use of a pharmaceutical formulation as described herein, in the manufacture of a medicament for use in the treatment of autoimmune diseases, chronic kidney disease (CKD), acute kidney injury (AKI), renal glomerular damage, nephritis, glomerulonephritis, interstitial nephritis, tubulointerstitial nephritis, diabetic nephropathy, cardiorenal syndrome (CRS), non-alcoholic steatohepatitis (NASH), non-alcoholic fatty liver disease (NAFLD), inflammatory bowel disease (IBD), Crohn's disease, colitis, ulcerative colitis, irritable bowel syndrome (IBS), rheumatoid arthritis, systemic lupus erythematosus, liver steatosis, liver fibrosis, gout, sickle cell disease, cystic fibrosis, vasculitis, anti-neutrophilic cytoplasmic autoantibody (ANCA)-associated vasculitis, asthma, chronic obstructive pulmonary disease (COPD), non-cystic fibrosis bronchiectasis (NCFB), vascular dysfunction, lipoprotein modification, or type 2 diabetes.

In some embodiments there is provided the use of a pharmaceutical formulation, as described herein, in the manufacture of a medicament for use in the treatment of coronary artery disease, acute coronary syndrome, heart failure, heart failure with reduced ejection fraction (HFrEF), heart failure with preserved ejection fraction (HFpEF), arrhythmia, cardiomyopathy, dilated cardiomyopathy, hypertrophic cardiomyopathy myocardial infarction, hypertension, pulmonary arterial hypertension (PAH), pulmonary hypertension, vascular dysfunction, atherosclerosis, ischemic heart disease, atrial fibrillation, pericarditis, diastolic dysfunction, atherosclerotic plaque rupture, abdominal aortic aneurysm, and/or chemotherapy-induced cardiotoxicity.

In some embodiments there is provided the use of a pharmaceutical formulation, as described herein, in the manufacture of a medicament for use in the treatment of Alzheimer's disease, Parkinson's disease, stroke, multiple sclerosis (MS), multiple system atrophy (MSA), amyotrophic lateral sclerosis (ALS), and /or epilepsy.

In some embodiments there is provided the use of a pharmaceutical formulation as described herein, in the manufacture of a medicament for the treatment of heart failure, heart failure with reduced ejection fraction, heart failure with preserved ejection fraction, non-alcoholic steatohepatitis (NASH), or chronic obstructive pulmonary disease (COPD), in a warm-blooded animal such as man.

In some embodiments there is provided the use of a pharmaceutical formulation as described herein, for use in the treatment of diseases or conditions in which modulation of the activity of the enzyme myeloperoxidase (MPO) is desirable.

In some embodiments there is provided the use of a pharmaceutical formulation as described herein, for use in the treatment of diseases or conditions in which inhibition of the activity of the enzyme myeloperoxidase (MPO) is desirable.

In some embodiments there is provided the use of a pharmaceutical formulation as described herein, for use in the treatment of diseases or conditions with inflammatory, cardiovascular, and/or neurological components, and/or neutrophilic driven diseases.

In some embodiments there is provided the use of a pharmaceutical formulation as described herein, for use in the treatment of autoimmune diseases, chronic kidney disease (CKD), acute kidney injury (AKI), renal glomerular damage, nephritis, glomerulonephritis, interstitial nephritis, tubulointerstitial nephritis, diabetic nephropathy, cardiorenal syndrome (CRS), non-alcoholic steatohepatitis (NASH), non-alcoholic fatty liver disease (NAFLD), inflammatory bowel disease (IBD), Crohn's disease, colitis, ulcerative colitis, irritable bowel syndrome (IBS), rheumatoid arthritis, systemic lupus erythematosus, liver steatosis, liver fibrosis, gout, sickle cell disease, cystic fibrosis, vasculitis, anti-neutrophilic cytoplasmic autoantibody (ANCA)-associated vasculitis, asthma, chronic obstructive pulmonary disease (COPD), non-cystic fibrosis bronchiectasis (NCFB), vascular dysfunction, lipoprotein modification, or type 2 diabetes.

In some embodiments there is provided the use of a pharmaceutical formulation as described herein, for use in the treatment of coronary artery disease, acute coronary syndrome, heart failure, heart failure with reduced ejection fraction (HFrEF), heart failure with preserved ejection fraction (HFpEF), arrhythmia, cardiomyopathy, dilated cardiomyopathy, hypertrophic cardiomyopathy myocardial infarction, hypertension, pulmonary arterial hypertension (PAH), pulmonary hypertension, vascular dysfunction, atherosclerosis, ischemic heart disease, atrial fibrillation, pericarditis, diastolic dysfunction, atherosclerotic plaque rupture, abdominal aortic aneurysm, and/or chemotherapy-induced cardiotoxicity.

In some embodiments there is provided the use of a pharmaceutical formulation as described herein, for use in the treatment of Alzheimer's disease, Parkinson's disease, stroke, multiple sclerosis (MS), multiple system atrophy (MSA), amyotrophic lateral sclerosis (ALS), and /or epilepsy.

In some embodiments there is provided the use of a pharmaceutical formulation as described herein, for use in the treatment of heart failure, heart failure with reduced ejection fraction, heart failure with preserved ejection fraction, non-alcoholic steatohepatitis (NASH), or chronic obstructive pulmonary disease (COPD)

As used herein, the terms "treatment" and "treat" refer to reversing, alleviating, delaying the onset of, or inhibiting the progress of a disease or disorder, or one or more symptoms thereof, as described herein. In some embodiments, treatment may be conducted after one or more symptoms have developed. In other embodiments, treatment may be conducted in the absence of symptoms. For example, treatment may be conducted to a susceptible individual prior to the onset of symptoms (e.g. in light of a history of symptoms and/or in light of genetic or other susceptibility factors). Treatment may also be continued after symptoms have resolved, for example to present or delay their recurrence.

### DETAILED DESCRIPTION OF THE FIGURES

Figure 1 depicts the direct compression process described herein.
Figure 2A compares the Compaction Pressure (CP) vs. Tensile Strength of Variants 1, 2, 3, and 14.
Figure 2B compares the Solid Fraction vs. Compaction Pressure (CP) of Variants 1, 2, 3, and 14.
Figure 2C compares the Solid Fraction vs. Tensile Strength (TS) of Variants 1, 2, 3, and 14.
Figure 3A compares the Compaction Pressure (CP) vs. Tensile Strength of Variants 5 and 10
Figure 3B compares the Solid Fraction vs. Compaction Pressure (CP) of Variants 5 and 10.
Figure 3C compares the Solid Fraction vs. Tensile Strength (TS) of Variants 5 and 10.
Figure 4A compares the Compaction Pressure (CP) vs. Tensile Strength of Variants 3, 7 and 11.
Figure 4B compares the Solid Fraction vs. Compaction Pressure (CP) of Variants 3, 7 and 11.
Figure 4C compares the Solid Fraction vs. Tensile Strength (TS) of Variants 3, 7 and 11.
Figure 5A compares the Compaction Pressure (CP) vs. Tensile Strength of Variants 9 and 12.
Figure 5B compares the Solid Fraction vs. Compaction Pressure (CP) of Variants 9 and 12.
Figure 5C compares the Solid Fraction vs. Tensile Strength (TS) of Variants 9 and 12.
Figure 6 compares the dissolution profiles of Variants 1, 2, and 3.
Figure 7 compares the dissolution profiles of Variants 5 and 10.
Figure 8 compares the dissolution profiles of Variants 3, 11, and 15.
Figure 9 compares the dissolution profiles of Variants 9, 12, and 15.
Figure 10A and 10B depict the dissolution profiles of Variant 15 before and after coating.
Figure 11 depicts the coating process described herein.
Figure 12 compares the dissolution profile of various film coated tablets coated at 2% w/w.
Figure 13 compares the dissolution profile of various film coated tablets coated at 4.5% w/w.
Figure 14 compares the dissolution profile of coated and uncoated tablets comprising 2.5 mg Compound 1 or 5 mg Compound 1.

### EXAMPLES

Compound 1 was produced by the synthesis route disclosed in WO2016/087338. The excipients used in the Example and Results sections are shown in Table 2.

**Table 2: Excipients, abbreviations and grades**

| **Excipient name** | **Abbreviation** | **Grades** | **/Supplier** |
|---|---|---|---|
| Mannitol | - | Pearlitol 100SD | Roquette |
| | | Pearlitol 200SD | |
| Microcrystalline cellulose | MCC | Avicel PH102 | DuPont |
| Dicalcium phosphate anhydrous | DCPA | Emcompress^{®} | JRS Pharma |
| Low-substituted hydroxypropyl cellulose | L-HPC | LH-31 | Shinetsu |
| | | LH-21 | |
| | | LH-B1 | |
| Crosscarmellose Sodium | - | Ac-Di-Sol | DuPont |
| Magnesium stearate | - | Ligamed MF-2-V | Peter Greven |

### Example 1: Manufacture of tablets

Tablets of Compound 1 as described herein were manufactured using a dry process with direct compression.

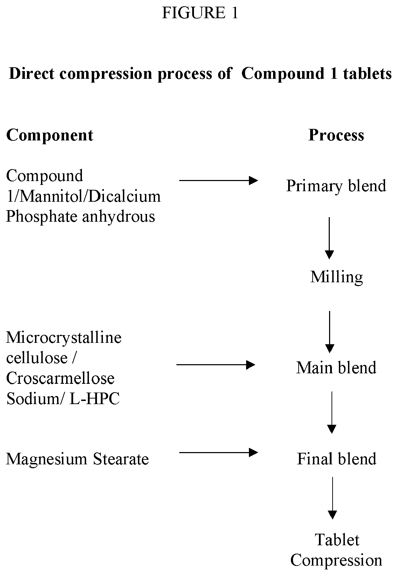

The formulations described in Tables 3-A and 3-B were prepared according (or analogous) to the procedure described below at a 1 kg scale and summarized in Figure 1. The values quoted in the table are %w/w.
1. Compound 1 was blended with mannitol, and if present, DCPA, in a ERWEKA DKM (Double Cone Mixer), 11L blender at approximately 200 revolutions at a speed of 20 rpm.
2. The resulting powder blend from step 1 was sieved through a 600µm screen at approximately 500rpm milling speed on a Frewitt mill CW-150.
3. The milled powder blend was subsequently blended with MCC, L-HPC, and/or croscarmellose sodium in in a ERWEKA DKM (Double Cone Mixer), 11L blender at approximately 200 revolutions at a speed of 20 rpm.
4. The resulting blend from step 3 was subsequently blended with magnesium stearate in in a ERWEKA DKM (Double Cone Mixer), 11L blender at approximately 200 revolutions at a speed of 20 rpm.
5. The tablet compression was performed on a Korsch XL-100 with appropriately sized oval or round tooling to prepare 400 mg (10 mg Compound 1), 200 mg (5 mg Compound 1), and 100 mg (2.5 mg Compound 1) tablets.

**Table 3-A**

| | Var. 1 | Var. 2 | Var. 3 | Var. 4* | Var. 5 | Var. 6* | Var. 7 | Var. 8* |
|---|---|---|---|---|---|---|---|---|
| Tablet Strength /mg | 10 | 10 | 10 | 10 | 10 | 10 | 10 | 10 |
| Compound 1 | 2.5 | 2.5 | 2.5 | 2.5 | 2.5 | 2.5 | 5.0 | 2.5 |

| Fillers | | | | | | | | |
|---|---|---|---|---|---|---|---|---|
| Mannitol, Pearlitol 100SD | 35.0 | 35.0 | 37.5 | 43.0 | 60.0 | 73.0 | 70.5 | *** |
| Mannitol, Pearlitol 200SD | *** | *** | *** | *** | *** | *** | *** | 73.0 |
| Dicalcium phosphate anhydrous | *** | *** | *** | *** | *** | *** | *** | *** |
| (DCPA), Emcompass | | | | | | | | |
| Microcrystalline cellulose, Avicel PH102 | 50.5 | 50.5 | 54.0 | 42.5 | 25.5 | 13.0 | 18.5 | 12.5 |

| Disintegrants | | | | | | | | |
|---|---|---|---|---|---|---|---|---|
| Low-substituted hydroxypropyl cellulose, LH-31 | 10.0 | *** | *** | 10.0 | 10.0 | 10.0 | *** | *** |
| Low-substituted hydroxypropyl cellulose, LH-21 | *** | 10.0 | *** | *** | *** | *** | *** | *** |
| Low-substituted hydroxypropyl cellulose, LH-B1 | *** | *** | *** | *** | *** | *** | *** | 10.0 |
| Croscarmellose Sodium | *** | *** | 4.0 | *** | *** | *** | 4.0 | *** |

| Lubricant | | | | | | | | |
|---|---|---|---|---|---|---|---|---|
| Magnesium Stearate | 2.0 | 2.0 | 2.0 | 2.0 | 2.0 | 1.5 | 2.0 | 2.0 |

| | | | | | | | | |
|---|---|---|---|---|---|---|---|---|
| * not executed | | | | | | | | |

**Table 3-B**

| | Var. 9 | Var. 10 | Var. 11A | Var. 11B | Var. 12 | Var. 13 | Var. 14 | Var. 15 | Var. 16 |
|---|---|---|---|---|---|---|---|---|---|
| Tablet Strength/ mg | 10 | 10 | 10 | 10 | 5 | 10 | 10 | 5 | 5 |
| Compound 1 | 2.5 | 2.5 | 2.5 | 2.5 | 2.5 | 5.0 | 5.0 | 2.5 | 2.5 |

| Fillers | | | | | | | | | |
|---|---|---|---|---|---|---|---|---|---|
| Mannitol, Pearlitol 100SD | 60.0 | 73.0 | 73.0 | 73.0 | *** | 53.0 | 58.0 | 55.0 | 60.0 |
| Mannitol, Pearlitol 200SD | *** | *** | *** | *** | *** | *** | *** | *** | *** |
| Dicalcium phosphate anhydrous, Emcompass | 10.0 | *** | *** | *** | 36.5 | *** | *** | *** | *** |
| Microcrystalline cellulose, Avicel PH102 | 15.5 | 12.5 | 18.5 | 18.5 | 55.0 | 36.0 | 25.0 | 36.5 | 25.5 |

| Disintegrants | | | | | | | | | |
|---|---|---|---|---|---|---|---|---|---|
| Low-substituted hydroxypropyl cellulose, LH-31 | 10.0 | *** | *** | *** | *** | *** | *** | *** | *** |
| Low-substituted hydroxypropyl cellulose, LH-21 | *** | *** | *** | *** | *** | *** | *** | *** | *** |
| Low-substituted hydroxypropyl cellulose, LH-B1 | *** | 10.0 | *** | *** | *** | *** | 10.0 | *** | 10.0 |
| Croscarmellose Sodium | *** | *** | 4.0 | 4.0 | 4.0 | 4.0 | *** | 4.0 | *** |

| Lubricant | | | | | | | | | |
|---|---|---|---|---|---|---|---|---|---|
| Magnesium Stearate | 2.0 | 2.0 | 2.0 | 2.0 | 2.0 | 2.0 | 2.0 | 2.0 | 2.0 |

### Example 2: Testing of Tablets

### Compression Profile

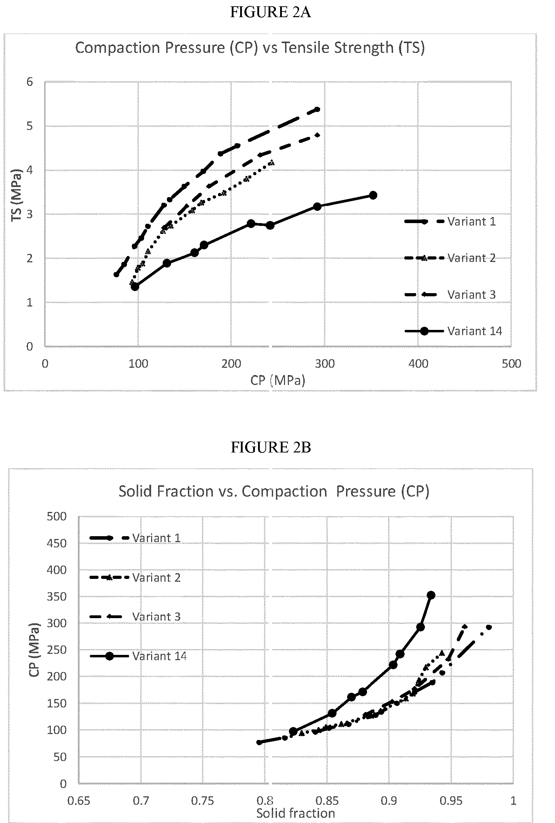

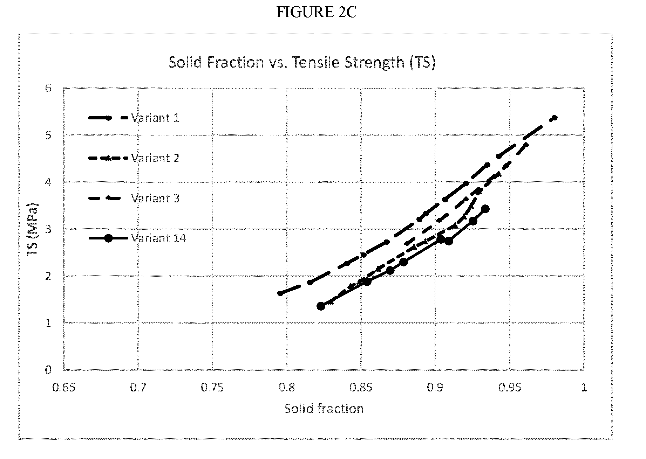

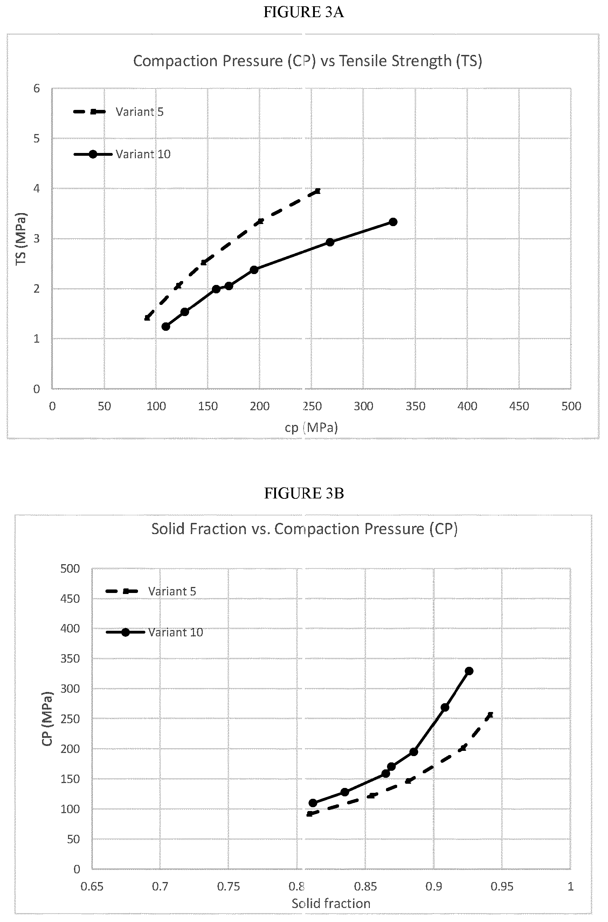

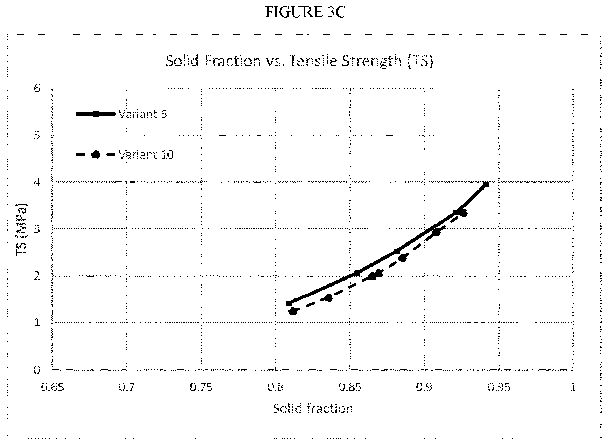

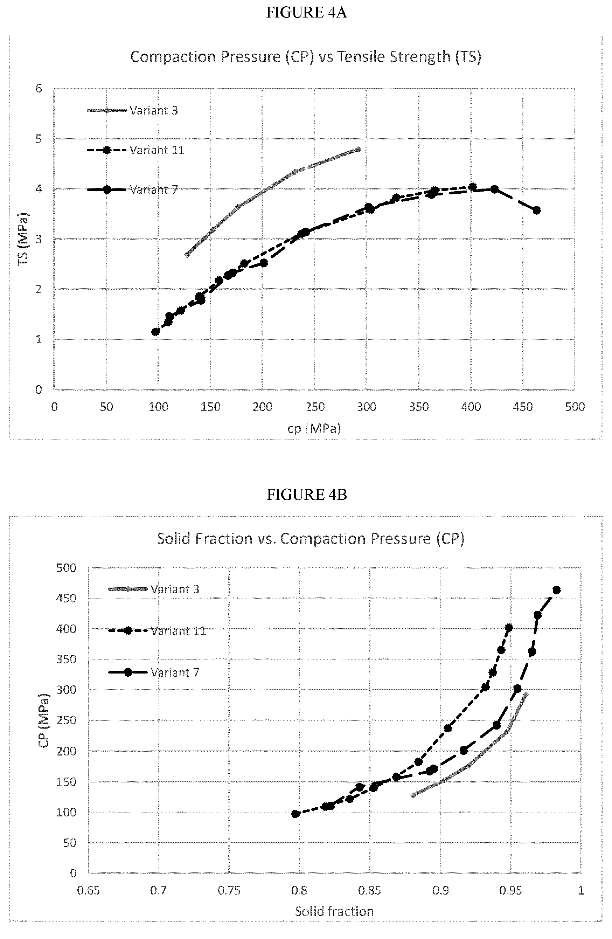

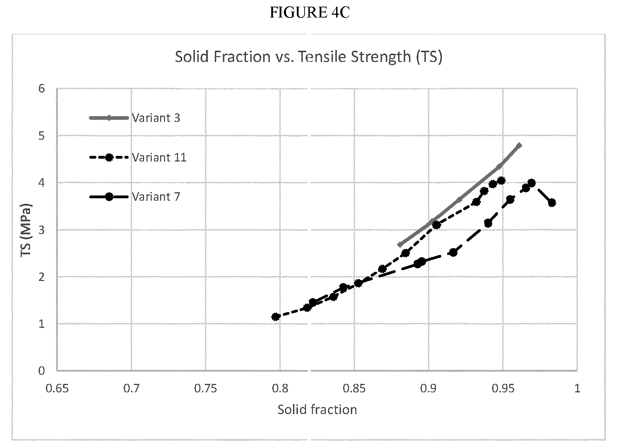

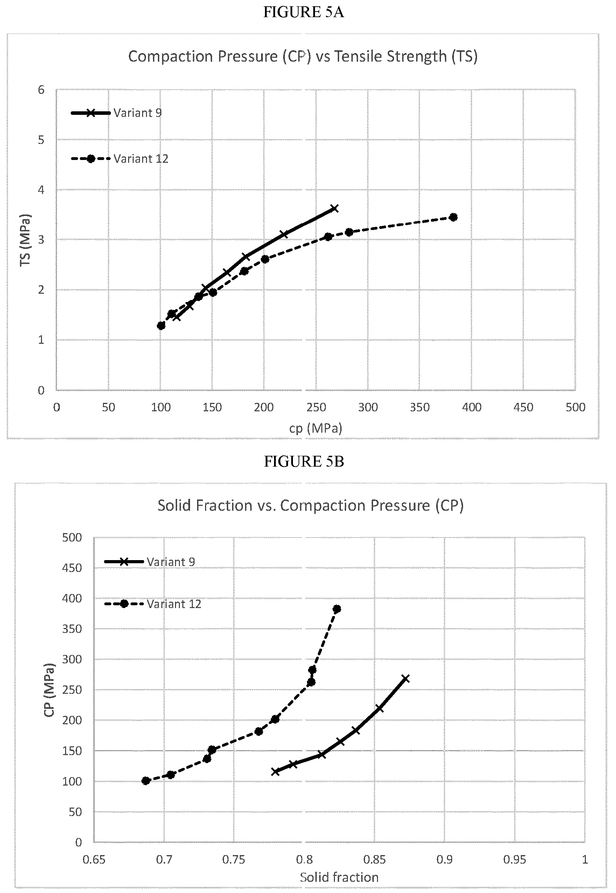

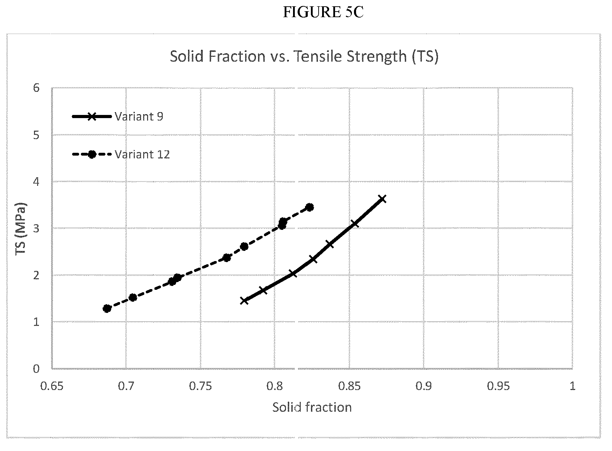

All batches were compressed separately using a suitable rotary tablet press (e.g. Korsch XL100) with the same punch design (round 6mm for 2.5mg strength; round 8mm for 5mg strength and oval 14mmx7mm for 10mg strength) under different compression forces. Breaking force and thickness of the produced tablets were characterized using suitable hardness and thickness measurement equipment. Tensile strength was calculated in terms of breaking force and dimension of produce tablets. The results are presented in Figures 2-5.

The compression profile depicts the tablet tensile strength as a function of the applied compaction force for all batches. This profile is normalised for the punch tip face area and tablet geometry, allowing for a true comparison of formulation robustness independent of tablet size.

### Dissolution Profiles

The dissolution profiles of the tablets were performed in accordance with the United States Pharmacopeia and the European Pharmacopoeia, using Apparatus II (paddle apparatus) with a stirring speed of 75 rpm in a pH 6.1 sodium phosphate (0.075 M)/citric acid (0.025 M) buffer. Concentration of Compound 1 is quantified by comparison of the UV response at 290 nm with a background correction at 400 nm of the dissolution sample with that of an external reference standard solution.

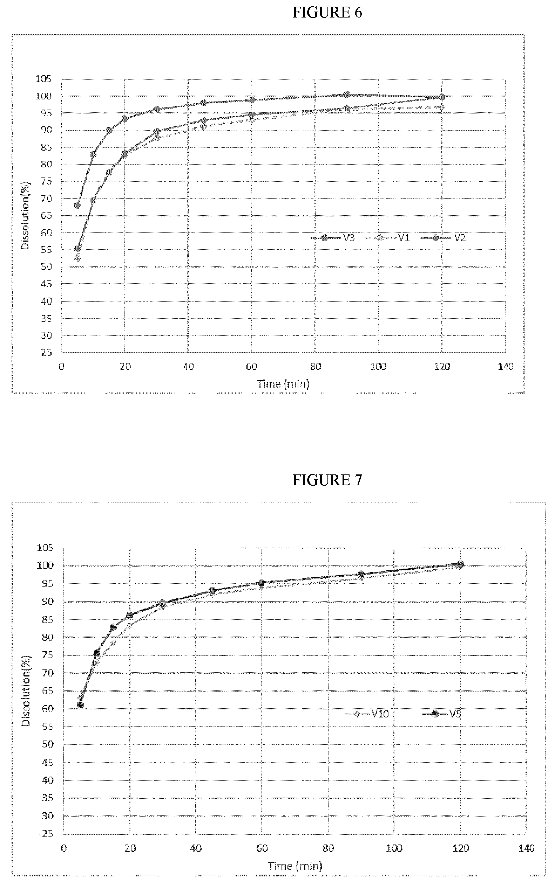

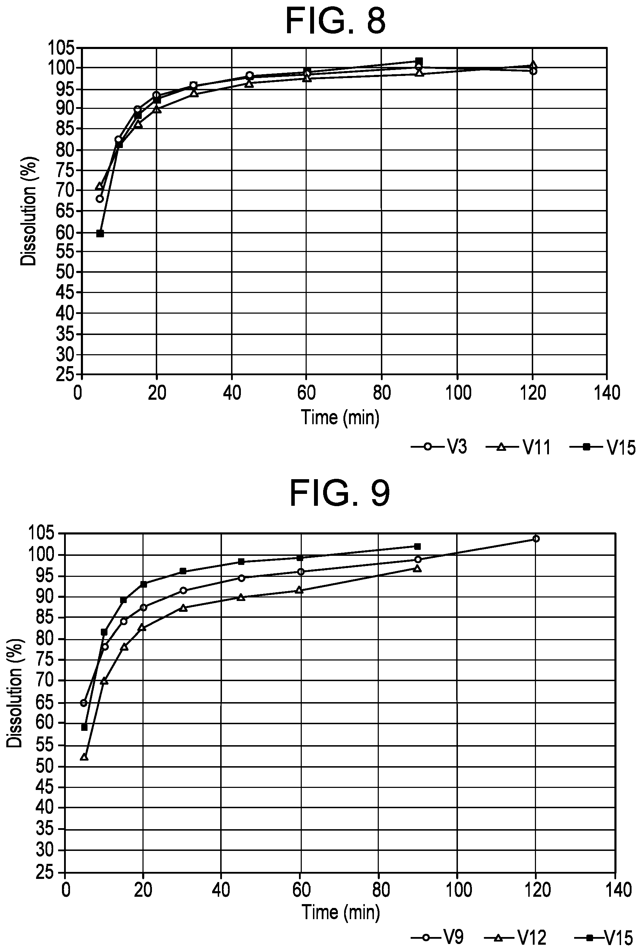

Dissolution profiles of the tablets were measured in pH6.1 USP medium with method 2. Figures 6-9 compare dissolution profiles of various formulations.

### Example 3: Coating

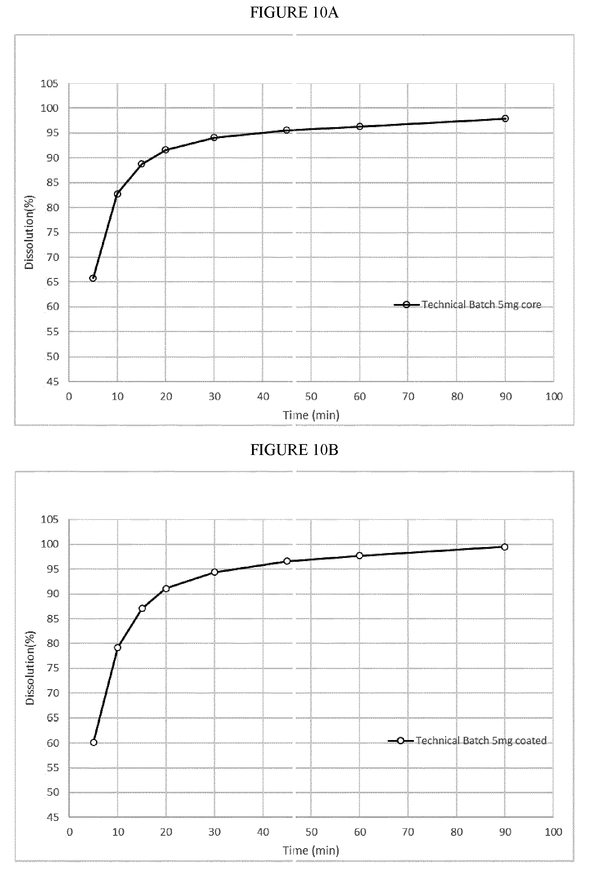

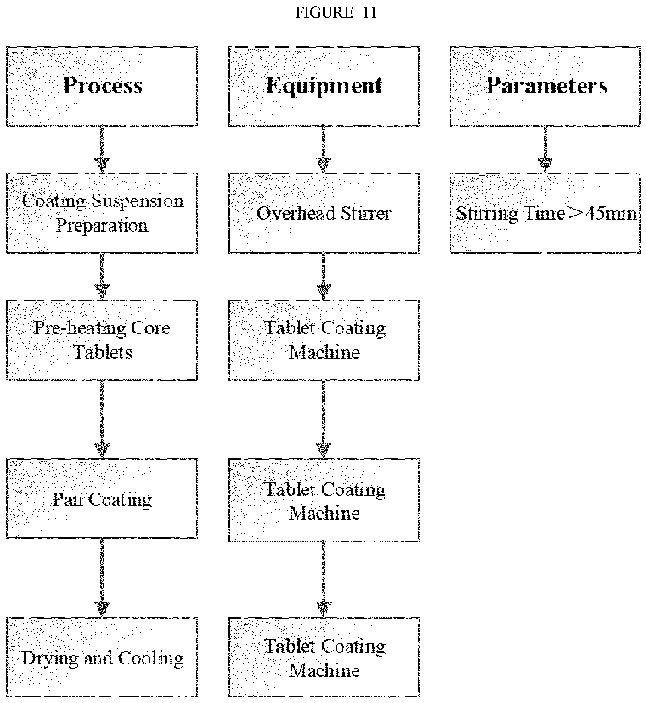

Tablets with the Variant 15 formulation comprising 2.5 mg of Compound 1 and 5mg of Compound 1 were film-coated using standard pan coating techniques as shown in Figure 11 with a HPMC/HPC coating (Aquarius Prime White, BKN318005, Ashland) for a weight gain of 3.5% w/w of the uncoated tablet for the 5mg tablet and 3.5% w/w of the uncoated tablet for the 2.5mg tablet. The dissolution profile of the 5mg coated tablet was measured in pH 6.1 USP medium with method 2 as described in Example 2. Figures 10A and 10B depict the dissolution profiles of the uncoated and coated tablet respectively.

Tablets comprising 2.5 mg Compound 1 (100 mg total weight, herein after "2.5 mg core tablets") and 5 mg Compound 1 (200 mg total weight, herein after "5 mg core tablets") having the composition of described in Table 4 were prepared according to Example 1.

**Table 4**

| **Component** | **w/w %** |
|---|---|
| Compound 1 | 2.5 |
| Mannitol, Pearlitol 100SD | 55 |
| Microcrystalline cellulose, Avicel PH102 | 36.5 |
| Croscarmellose Sodium | 4 |
| Magnesium Stearate | 2 |

The 5 mg core tablets were film-coated using standard pan coating techniques as shown in Figure 11 with aqueous suspensions of the proprietary coatings listed in Table 5 for weight gains of 2% or 4.5% w/w.

**Table 5**

| Main Polymer(s) | Coating | Conc. % (w/w) | Manufacturer |
|---|---|---|---|
| HPMC, PVPP, HPC (with plasticizer) | Aquarius^{™} Preferred HSP BON313109 Orange | 20 | Ashland |
| PVA (no plasticizer) | Opadry^{™} 85A630003-CN Orange | 20 | Colorcon |
| HPMC (with plasticizer) | Opadry^{™} 03K630008-CN Orange | 15 | Colorcon |
| PVA (with plasticizer) | Opadry^{™} II 85F630054-CN Orange | 20 | Colorcon |

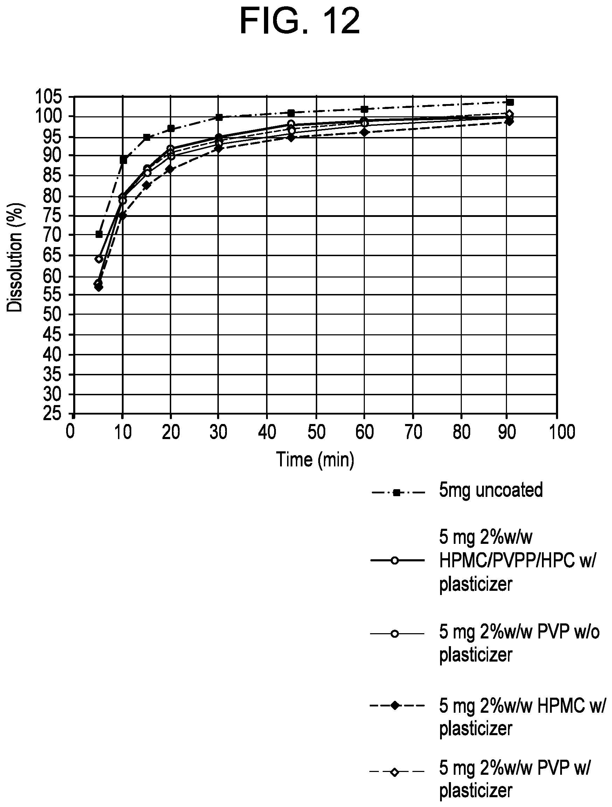

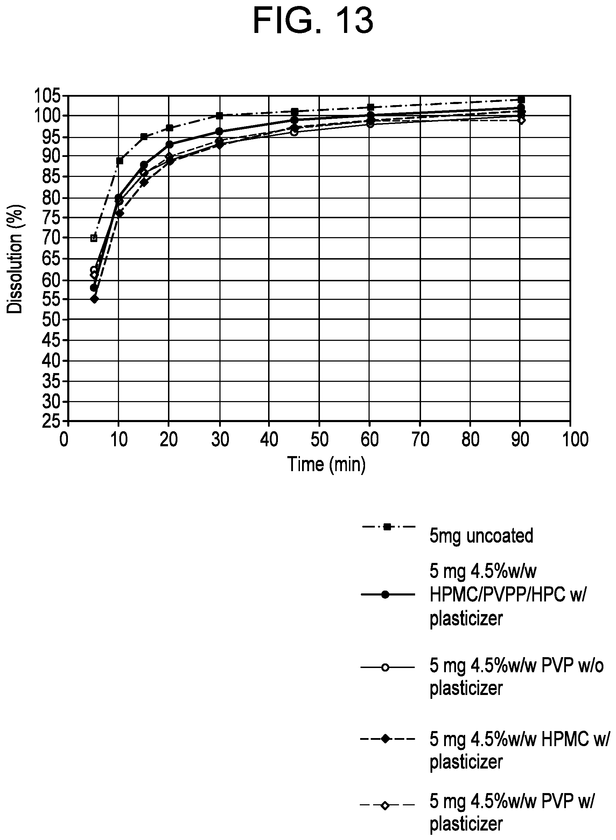

Dissolution profiles of the tablets were measured in pH 6.1 USP medium with method 2 as described in Example 2. Figures 12 and 13 compare dissolution profiles of the 2% w/w coatings and 4.5% coatings respectively.

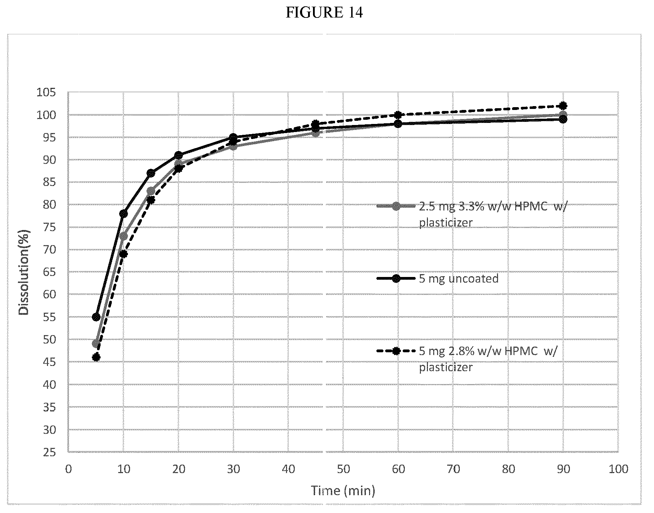

The 2.5 mg core tablets were coated with Opadry 03K630008-CN Orange for a weight gain of 3.3% and the 5 mg core tablets were coated with Opadry 03K630008-CN Orange for a weight gain of 2.8%. Dissolution profiles of the tablets were measured in pH 6.1 USP medium with method 2 as described in Example 2 and the results are shown in Figure 14.

## Claims

1. A pharmaceutical formulation comprising Compound 1, one or more pharmaceutical fillers, one or more pharmaceutical disintegrants, and one or more pharmaceutical lubricant, wherein Compound 1 is 1-{2-[(1R)-1-aminoethyl]-4-chlorobenzyl}-2-thioxo-1,2,3,5-tetrahydro-4H-pyrrolo[3,2-d]pyrimidin-4-one

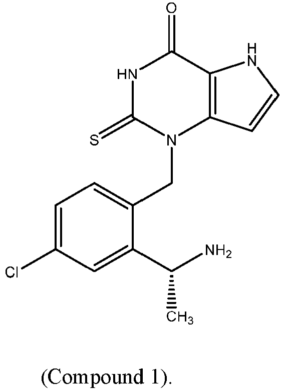

2. The pharmaceutical formulation of claim 1, wherein one of the pharmaceutical disintegrants is croscarmellose sodium.

3. The pharmaceutical formulation of claim 2, comprising 4% w/w croscarmellose sodium.

4. The pharmaceutical formulation of any of claims 1 to 3, wherein one of the pharmaceutical lubricant is magnesium stearate.

5. The pharmaceutical formulation of claim 4 comprising 2% w/w magnesium stearate.

6. The pharmaceutical formulation of any of claims 1 to 5 wherein one of the pharmaceutical fillers is mannitol.

7. The pharmaceutical formulation of claim 6 comprising 55% w/w mannitol.

8. The pharmaceutical formulation of any of claims 1-7 wherein one of the pharmaceutical fillers is microcrystalline cellulose.

9. The pharmaceutical formulation of claim 8 comprising 36.5% w/w microcrystalline cellulose.

10. A pharmaceutical formulation of claim 1 comprising Compound 1,
wherein one of the pharmaceutical fillers is mannitol,
wherein one of the pharmaceutical fillers is microcrystalline cellulose,
wherein one of the pharmaceutical disintegrants is croscarmellose sodium, and
wherein one of the pharmaceutical lubricant is magnesium stearate.

11. The pharmaceutical formulation of claim 10 wherein Compound 1 is present at about 2.5% w/w, the mannitol is present at about 55% w/w, the microcrystalline cellulose is present at about 36.5% w/w, the croscarmellose sodium is present at about 4% w/w, and the magnesium stearate is present at about 2% w/w.

12. The pharmaceutical formulation of any of claims 1-11 comprising 5 mg of Compound 1.

13. The pharmaceutical formulation of any of claims 1-12, wherein the pharmaceutical formulation is a tablet.

14. The pharmaceutical formulation of claim 13, wherein the tablet further comprises a film-coating.

15. A pharmaceutical formulation of any one of claims 12 -14 for use in the treatment of heart failure, heart failure with reduced ejection fraction, heart failure with preserved ejection fraction, non-alcoholic steatohepatitis (NASH), or chronic obstructive pulmonary disease (COPD).

## Patentansprüche

1. Pharmazeutische Formulierung, die Verbindung 1 umfasst, einen oder mehrere pharmazeutische Füllstoffe, ein oder mehrere pharmazeutische Sprengmittel und ein oder mehrere pharmazeutische Gleitmittel, wobei Verbindung 1 1-{2-[(1R)-1-Aminoethyl]-4-chlorbenzyl}-2-thioxo-1,2,3,5-tetrahydro-4H-pyrrolo[3,2-d]pyrimidin-4-on ist

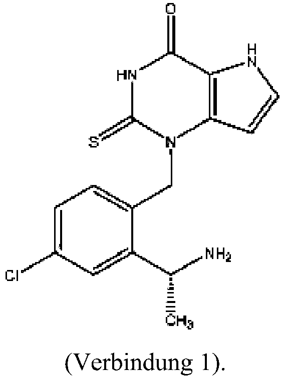

2. Pharmazeutische Formulierung nach Anspruch 1, wobei eines der pharmazeutischen Sprengmittel Croscarmellose-Natrium ist.

3. Pharmazeutische Formulierung nach Anspruch 2, die 4 Gew.-% Croscarmellose-Natrium umfasst.

4. Pharmazeutische Formulierung nach einem der Ansprüche 1 bis 3, wobei eines der pharmazeutischen Gleitmittel Magnesiumstearat ist.

5. Pharmazeutische Formulierung nach Anspruch 4, die 2 Gew.-% Magnesiumstearat umfasst.

6. Pharmazeutische Formulierung nach einem der Ansprüche 1 bis 5, wobei einer der pharmazeutischen Füllstoffe Mannit ist.

7. Pharmazeutische Formulierung nach Anspruch 6, die 55 Gew.-% Mannitol umfasst.

8. Pharmazeutische Formulierung nach einem der Ansprüche 1-7, wobei einer der pharmazeutischen Füllstoffe mikrokristalline Cellulose ist.

9. Pharmazeutische Formulierung nach Anspruch 8, die 36,5 Gew.-% mikrokristalline Cellulose umfasst.

10. Pharmazeutische Formulierung nach Anspruch 1, die Verbindung 1 umfasst,
wobei einer der pharmazeutischen Füllstoffe Mannit ist,
wobei einer der pharmazeutischen Füllstoffe mikrokristalline Cellulose ist, wobei eines der pharmazeutischen Sprengmittel Croscarmellose-Natrium ist, und wobei eines der pharmazeutischen Gleitmittel Magnesiumstearat ist.

11. Pharmazeutische Formulierung nach Anspruch 10, wobei die Verbindung 1 zu etwa 2,5 Gew.-% vorliegt, das Mannitol zu etwa 55 Gew.-% vorliegt, die mikrokristalline Cellulose zu etwa 36,5 Gew.-% vorliegt, das Croscarmellose-Natrium zu etwa 4 Gew.-% vorliegt und das Magnesiumstearat zu etwa 2 Gew.-% vorliegt.

12. Pharmazeutische Formulierung nach einem der Ansprüche 1-11, die 5 mg von Verbindung 1 umfasst.

13. Pharmazeutische Formulierung nach einem der Ansprüche 1-12, wobei die pharmazeutische Formulierung eine Tablette ist.

14. Pharmazeutische Formulierung nach Anspruch 13, wobei die Tablette ferner eine Filmbeschichtung aufweist.

15. Pharmazeutische Formulierung nach einem der Ansprüche 12-14 zur Verwendung bei der Behandlung von Herzinsuffizienz, Herzinsuffizienz mit reduzierter Ejektionsfraktion, Herzinsuffizienz mit erhaltener Ejektionsfraktion, nicht-alkoholischer Steatohepatitis (NASH) oder chronisch obstruktiver Lungenerkrankung (Chronic Obstructive Pulmonary Disease, COPD).

## Revendications

1. Préparation pharmaceutique comprenant le composé 1, une ou plusieurs charges pharmaceutiques, un ou plusieurs délitants pharmaceutiques, et un ou plusieurs lubrifiants pharmaceutiques, dans laquelle le composé 1 est 1-{2-[(1R)-1-aminoéthyl]-4-chlorobenzyl}-2-thioxo-1,2,3,5-tétrahydro-4H-pyrrolo[3,2-d]pyrimidin-4-one

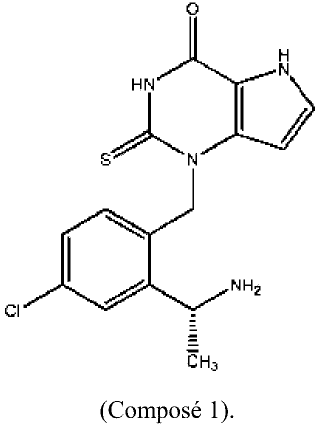

2. Préparation pharmaceutique de la revendication 1, dans laquelle un des délitants pharmaceutiques est de la croscarmellose sodique.

3. Préparation pharmaceutique de la revendication 2, comprenant 4 % p/p de croscarmellose sodique.

4. Préparation pharmaceutique de quelconques des revendications 1 à 3, dans laquelle un des lubrifiants pharmaceutiques est du stéarate de magnésium.

5. Préparation pharmaceutique de la revendication 4, comprenant 2 % p/p de stéarate de magnésium.

6. Préparation pharmaceutique de quelconques des revendications 1 à 5, dans laquelle une des charges pharmaceutiques est du mannitol.

7. Préparation pharmaceutique de la revendication 6, comprenant 55 % p/p de mannitol.

8. Préparation pharmaceutique de quelconques des revendications 1 à 7, dans laquelle une des charges pharmaceutiques est de la cellulose microcristalline.

9. Préparation pharmaceutique de la revendication 8, comprenant 36,5 % p/p de cellulose microcristalline.

10. Préparation pharmaceutique de la revendication 1, comprenant le composé 1,
dans laquelle une des charges pharmaceutiques est du mannitol,
dans laquelle une des charges pharmaceutiques est de la cellulose microcristalline,
dans laquelle un des délitants pharmaceutiques est de la croscarmellose sodique, et
dans laquelle un des lubrifiants pharmaceutiques est du stéarate de magnésium.

11. Préparation pharmaceutique de la revendication 10, dans laquelle le composé 1 est présent à environ 2,5 % p/p, le mannitol est présent à environ 55 % p/p, la cellulose microcristalline est présente à environ 36,5 % p/p, la croscarmellose sodique est présente à environ 4 % p/p, et le stéarate de magnésium est présent à environ 2 % p/p.

12. Préparation pharmaceutique de quelconques des revendications 1 à 11, comprenant 5 mg du composé 1.

13. Préparation pharmaceutique de quelconques des revendications 1 à 12, dans laquelle la préparation pharmaceutique est un comprimé.

14. Préparation pharmaceutique de la revendication 13, dans laquelle le comprimé comprend en outre un revêtement-film.

15. Préparation pharmaceutique de l'une quelconque des revendications 12 à 14 pour l'utilisation dans le traitement de : insuffisance cardiaque, insuffisance cardiaque avec fraction d'éjection réduite, insuffisance cardiaque avec fraction d'éjection préservée, stéatohépatite non alcoolique (NASH), ou maladie pulmonaire obstructive chronique (MPOC).
